# EUROPÄISCHE PATENTSCHRIFT

(11) **EP 0 657 106 B1**
(45) Veröffentlichungstag und Bekanntmachung des Hinweises auf die Patenterteilung: **09.08.2000**
(21) Anmeldenummer: 94117312.2
(22) Anmeldetag: 03.11.1994
(51) Int. Cl.: A23L 1/236, C08B 37/00, C07H 3/06

(54) **Hydrierte Fructooligosaccharide**
Hydrogenated fructooligosaccarides
Fructooligo saccharides hydrogénés

(30) Priorität: 08.12.1993 DE 4341780
(43) Veröffentlichungstag der Anmeldung: 14.06.1995
(73) Patentinhaber: SÜDZUCKER AKTIENGESELLSCHAFT MANNHEIM/OCHSENFURT, D-68165 Mannheim (DE)
(72) Erfinder: Vogel, Manfred, Dr., D-67271 Neuleiningen (DE); Kunz, Markwart, Dr., D-67550 Wroms (DE); Kowalczyk, Jörg, Dr., D-67269 Grünstadt (DE); Munir, Mohammed, Dr., D-67271 Kindenheim (DE)
(74) Vertreter: Gleiss, Alf-Olav, Dr.jur. Dipl.-Ing.

(56) Entgegenhaltungen:
- EP-A- 0 440 074
- EP-A- 0 561 088
- WO-A-93/02566
- GB-A- 2 072 679
- GB-A- 2 105 338
- FALBE ET AL.: "Römpp-Chemie-Lexikon, Bd. 3, 9. Auflage" 1, 1990, GEORG THIEME VERLAG, STUTTGART
- BEYER ET AL.: "Lehrbuch der Organischen Chemie, 20. Auflage" 2, 1984, S. HIRZEL VERLAG, STUTTGART

## Beschreibung

Gegenstand der Erfindung sind neue, als Süßungsmittel geeignete hydrierte Fructooligosaccharide vom Typ (Fructosyl)ₙ-Mannit und (Fructosyl)ₙ-Sorbit, sowie Verfahren zu ihrer Herstellung.

Die zunehmend bewußter werdende Ernährung stellt einige spezielle Anforderungen an Lebensmittel insbesondere jedoch an Süßungsmittel. Diese sind:
1. Nicht-kariogenität; das Süßungsmittel muß zahnschonend sein. Es darf durch die im Mund vorhandenen Mikroorganismen weder zu Säuren noch zu plaguebildenden Polysacchariden umgewandelt werden können.
2. Diabetikereignung; das Süßungsmittel darf beim Verzehr keine überhöhte Insulinsekretion verursachen. Es ist sogar wünschenswert, daß es durch Hemmung der Enzymtätigkeit in Dünndarm die Resorption anderer Nahrungsbestandteile verlangsamt und dadurch zur Vermeidung von Insulinspitzen führt.
3. Verminderte kalorische Nutzung; das Süßungsmittel soll vom menschlichen Organismus nicht verstoffwechselt werden können. Es ist zusätzlich vorteilhaft, wenn das Süßungsmittel durch Hemmung der Enzymtätigkeit in Dünndarm die Resorption anderer Nahrungsbestandteile verlangsamt und dadurch zu einer insgesamt geringeren Kalorienaufnahme führt.
4. Das Süßungsmittel soll im Dickdarmbereich der Vermehrung von bifidogenen Mikroorganismen dienen.
5. Das Süßungsmittel soll einen rein süßen Geschmack haben und sich in Lebensmitteln unter den unterschiedlichsten Bedingungen (z.B. weiter Temperatur- bzw. pH-Bereich) verarbeiten lassen.

Die dem Menschen seit dem Altertum bekannte Saccharose erfüllt nur die zuletzt genannte Forderung. Es wird deshalb angestrebt, alternative Süßungsmittel zu finden, die auch die anderen Forderungen erfüllen.

Es ist bekannt, Xylit als diabetiker-geeignetes, nichtkariogenes Süßungsmittel zu verwenden (A. Bär im Alternative Sweeteners, L.O'B. Nabors und R.C. Gelardi (ed.), Marcel Dekker, New York, 1986). Es hat jedoch mit 4 kcal/g den gleichen Nährwert wie Saccharose. Durch die hohe endothermische Lösungswärme ist die Verwendung von Xylit in einer Reihe von Lebensmitteln problematisch. Außerdem kann Xylit in Backwaren und Hartkaramellen nur mit großen Einschränkungen eingesetzt werden. Ein weiterer Nachteil von Xylit ist der für ein Süßungsmittel relativ hohe Preis.

Ein weiteres zahnschonendes und diabetiker-geeignetes Süßungsmittel ist der Sorbit (B.K. Dwivedi in Alternative Sweeteners, L.O'B. Nabors und R.C. Gelardi (ed.), Marcel Dekker, New York, 1986). Die unter Xylit aufgezählten Nachteile gelten gleichermaßen auch für Sorbit (mit Ausnahme des Preises der nicht so hoch ist). Auch hydrierte Stärkehydrolysate sind als alternative Süßungsmittel bekannt (B.K. Dwivedi in Alternative Sweeteners, L.O'B. Nabors und R.C. Gelardi (ed.), Marcel Dekker, New York, 1986). Diese können im Dünndarm hydrolysiert werden und da sie dabei Glucose freisetzen, sind sie für Diabetiker ungeeignet. Auch sonst ist deren Verwendungsmöglichkeit in Lebensmitteln aufgrund ungünstiger physikalischen Eigenschaften relativ eingeschränkt.

Kristalline Fructose ist ein anderes Süßungsmittel mit interessanten physiologischen Eigenschaften (T.F. Osberger im Alternative Sweeteners, L.O'B. Nabors und R.C. Gelardi (ed.), Marcel Dekker, New York, 1986), die jedoch auch gravierende Nachteile zeigt. Die Süßkraft der Fructose ist abhängig von der Temperatur, wodurch korrekte Dosierung in Lebensmitteln problematisch wird. Durch die geringe Thermostabilität der Fructose ist deren Anwendung in Lebensmitteln die gekocht bzw. gebacken werden, nur mit großen Einschränkungen möglich.

WO-A-93 02566 beschreibt die Verwendung von aus Topinambur- oder Zichorien-Inulin hergestellten Fructooligosacchariden zur Schokoladenherstellung, wobei auch die Möglichkeit erwähnt wird, die erhaltenen homo- und heterooligomeren Fructooligosaccharide zu hydrieren.

Isomalt, ein zahn-freundliches, diabetiker-geeignetes und minderkalorisches Süßungsmittel (P.J. Sträter in Alternative Sweeteners, L.O'B. Nabors und R.C. Gelardi (ed.), Marcel Dekker, New York, 1986) hat eine im Vergleich zu Saccharose zu niedrige Löslichkeit in Wasser, wodurch es zu Auskristallisation in Lebensmitteln mit hohem Trockensubstanzgehalt (z.B. Konfitüren) kommt.

Die Erfindung hat nun die Aufgabe, ein Süßungsmittel bereitzustellen, das
1) einen der Saccharose vergleichbaren Süßgeschmack aufweist,
2) sich in Lebensmitteln gut verarbeiten läßt,
3) weder Zahnkaries noch Plaque-Bildung verursacht bzw. fördert,
4) beim Verzehr keine überhöhten Insulinwerte im Blut verursacht,
5) selbst nicht oder nur zum Teil kalorisch verwertbar ist,
6) die Enzymkomplexe der Dünndarm-Mucosa hemmt und dadurch die kalorische Verwertung anderer Kohlenhydrate herabsetzt,
7) im Dickdarmbereich die Vermehrung von bifidogenen Mikroorganismen fördert und
8) leicht und preiswert aus natürlichen Quellen zugänglich ist.

Diese Aufgabe wird durch die Merkmale der Ansprüche 1 und 2 gelöst und durch die Merkmale der Unteransprüche gefördert.

Das erfindungsgemäß bereitgestellte, aus hydrierten Fructooligosacchariden vom Typ Fₙ-Mannit und/oder Fₙ-Sorbit (n = 1-6) bestehende Süßungsmittel ist leicht aus einem langkettigen Inulin durch Hydrolyse zu kürzerkettigen Polyfructosiden, chromatographische Abtrennung der verbleibenden langkettigen und Glucose-haltigen Produkte und Hydrierung erhältlich. Es hat einen angenehmen reinen Süßgeschmack. Die Süßkraft beträgt ca. 0,45 im Vergleich zu Saccharose (Süßkraft = 1,0).

Das erfindungsgemäße Süßungsmittel wird von den im Mund vorkommenden Streptokokken (S. mutans) weder zu Säure- noch zu Plaque-Bildung verwertet. Damit ist es als zahnschonend einzustufen.

Weder die Fructosylmannit- noch die Fructosylsorbitbindung wird von dem Enzymkomplex der Dünndarmmucosa mit nennenswerter Rate gespalten, so daß das erfindungsgemäße Süßungsmittel nicht resorbiert werden kann.

Die beim Verzehr von saccharosehaltigen Lebensmitteln auftretende hohe Insulinkonzentration im Blut wird beim Einsatz des erfindungsgemäßen Süßungsmittels nicht beobachtet. Damit ist es für die Diabetikerernährung gut geeignet.

Ein besonderer Vorteil des erfindungsgemäßen Süßungsmittels ist seine Wirkung auf die Darmflora. Tests mit Versuchspersonen zeigten eine deutliche Zunahme von Bifidobacterien im Stuhl. Somit ist das erfindungsgemäße Süßungsmittel geeignet, die Zusammensetzung der Darmflora zugunsten der für die Menschen vorteilhaften bifidogenen Mikroorganismen zu verschieben.

Für die Herstellung des erfindungsgemäßen Süßungsmittels startet man zweckmäßigerweise von einem langkettigen Inulin. Auch ein geeignetes Inulinhydrolysat (z.B. Raftilose® L75 der Firma Raffinerie Tirlemontoise, Tienen, Belgien) kann als Rohstoff eingesetzt werden.

Hohe Gehalte an Inulin sind in Topinambur- und Dahlienknollen sowie in Zichorienwurzeln zu finden. Die Kettenlänge von Inulin hängt sowohl von der Wachstumsphase der Pflanzen als auch von der Pflanzenart ab. So geben R.H.F. Beck und W. Praznik (Stärke 38 (1986) 391-394) einen durchschnittlichen Polymerisationsgrad von 20 für Dahlien-Inulin, 11 für Zichorien-Inulin und 5 für Topinambur-Inulin an.

Inuline sind nicht-reduzierende Heterofructane, die an einer β-2-1- verknüpften Kette von Fructofuranose-Molekülen als Abschluß eine α-D-Glucose am reduzierenden Ende tragen. Neben diesen Heterofructanen sind in wässrigen Extrakten von inulin-haltigem Pflanzenmaterial auch Spuren von Oligosacchariden nachweisbar, die nur β-2-1-verknüpfte Fructofuranose-Moleküle enthalten, wobei das letzte Fructosemolekül reduzierend ist. Der Anteil dieser homooligomeren Fructosemoleküle läßt sich erhöhen, wenn wie in JP 61-40754 beschrieben, durch Einwirkung einer Säure wie z. B. Oxalsäure auf Inulin ein Oligosacharidgemisch entsteht, das hauptsächlich kurzkettige Oligosaccharide enthält. Hier ist auch Inulobiose und Inulotriose nachgewiesen worden.

Wie der DE 40 03 140-A1 zu entnehmen ist, lassen sich Gemische von hetero- und homooligomeren Fructooligosacchariden mit Kettenlängen bis DP 7 erhalten, wenn Rohinulin enzymatisch unter geeigneten Bedingungen mit einer endo-Inulinase behandelt wird. Der Gehalt an homooligomeren Fructooligosacchariden, die allein für die Herstellung des erfindungsgemäßen Süßungsmittels in Frage kommen, ist bei dieser Arbeitsweise zu niedrig.

Behandelt man hingegen langkettiges Inulin mit einer mittleren Kettenlänge von DP ≥20, das entsprechend der deutschen Patentanmeldung P 43 16 425.0 aus Rohinulin gewonnenen werden kann, mit einer endo-Inulinase, dann erhält man ein Produktgemisch das mehr als 80 % an den gewünschten homooligomeren Fructooligosacchariden enthält.

Ein handelsübliches Inulinhydrolysat mit der Handelsbezeichnung Raftilose® L75 (Hersteller: Raffinerie Tirlemontoise, Tienen, Belgien) enthält mehr als 48 % der Trockensubstanzgehalt an kurzkettigen Fructooligosacchariden vom Typ Fₙ (mit n = 2-7) neben 15 - 17 % an Glucose, Fructose und Saccharose. Der Rest besteht aus kurzkettigen Oligosacchariden vom Typ Glucosylfructose (G-Fₙ mit n = 1-6). Auch dieses Produkt kann als Ausgangsstoff für die Gewinnung von homooligomeren Fructooligosacchariden, die für die Herstellung des erfindungsgemäßen Süßungsmittels benötigt werden, eingesetzt werden.

Langkettiges Inulin z. B. hergestellt entsprechend der deutschen Patentanmeldung P 43 16 425.0 ist der bevorzugte Rohstoff für die Herstellung von homooligomeren Fructooligosacchariden und somit auch für die Herstellung der erfindungsgemäßen Süßungsmittel.

Ausgehend von langkettigem Inulin läßt sich das erfindungsgemäße Süßungsmittel wie folgt herstellen:

Eine wässrige Lösung des langkettigen Inulins wird unter geeigneten Bedingungen enzymatisch mit einer endo-Inulinase behandelt. Dabei wird das Inulin partiell hydrolysiert und man erhält ein Produktgemisch das zu mehr als 80 % aus homooligomeren Fructooligosacchariden vom Typ Fₙ (n = 2-7) besteht. Der Rest sind im wesentlichen Fructose neben kleineren Mengen an Heterooligosacchariden vom Typ G-Fₙ (n = 1-6), die sich leicht durch chromatographische Trennung an Ca⁺⁺-beladenen, starksauren Kationenaustauscherharzen abtrennen lassen, so daß man ein nur homooligomere Fructooligosaccharide enthaltendes Produkt bekommt.

Dieses Produkt wird nun mit Wasserstoff nach einer der an sich bekannten Methoden hydriert. Das so erhaltene hydrierte, aus einem Gemisch aus (Fructosyl)ₙ-Mannit und (Fructosyl)ₙ-Sorbit (n = 1-6) bestehende Produkt stellt ein Gemisch der erfindungsgemäßen Süßungsmittel dar.

### Beispiel 1: Herstellung aus langkettigem Inulin

1 kg langkettiges Inulin (mittlere Kettenlänge DP 24,8) wurde in 30 l einer 50 mM Na-Acetat Pufferlösung, pH 5,3 eingerührt und bei 85 °C für 20 Minuten erhitzt. Die so entstandene klare Lösung wurde auf 54 °C abgekühlt und mit 2000 Einheiten endo-Inulinase (Produktbezeichnung "SP 168", erhältlich von NOVO Nordisk, Kopenhagen) versetzt. Der Ansatz wird für 48 h unter langsamem Rühren bei 54 °C gehalten. Danach wurde die Reaktion durch Inaktivierung des Enzyms durch pH-Anhebung auf 8,5 - 9,0 bzw. durch 20-minütiges Erhitzen auf 95 °C gestoppt. Nach HPLC-Analyse besteht das Produkt zu 80 % auf Trockensubstanzgehalt aus homooligomeren Fructooligosacchariden mit DP 2-7. Der Rest sind heterooligomere Fructooligosaccharide sowie freie Fructose.

Die weitere Behandlung des Produkts durch Chromatographie und Hydrierung wird in den nachfolgenden Beispielen 3 bzw. 4 näher erläutert.

### Beispiel 2: Herstellung aus langkettigem Inulin

1 kg langkettiges Inulin (mittlere Kettenlänge DP 29,5) wurde in 30 l einer 50 mM Na-Acetat Pufferlösung,pH 5,3 eingerührt und durch 20-minütiges Erhitzen auf 85 °C gelöst. Die Lösung wurde auf 54 °C abgekühlt und mit 2.000 Einheiten endo-Inulinase (Produktbezeichnung "SP 168", erhältlich vom NOVO Nordisk, Kopenhagen) versetzt. Der Ansatz wurde für 48 h unter langsamem Rühren bei 54 °C gehalten. Danach wurde die Reaktion durch pH-Anhebung auf 8,5 - 9,0 bzw. durch 20-minütiges Erhitzen auf 95 °C beendet. Eine HPLC-Analyse zeigt, daß das Produkt 83 % auf Trockensubstanzgehalt homooligomere Fructooligosaccharide mit DP 2-7 enthält. Der Rest sind heterooligomere Fructooligosaccharide und freie Fructose.

Die weitere Behandlung des Produkts durch Chromatographie und Hydrierung wird in den nachfolgenden Beispielen 3 bzw. 4 näher erläutert.

### Beispiel 3: Chromatographische Trennung

Eine Trennsäule mit 0,25 m Innendurchmesser und 10 m Länge wird mit einem Ca⁺⁺-beladenen schwachvernetzten starksauren Kationenaustauscherharz (z.B. Duolite® C 204) gefüllt und auf eine Temperatur zwischen 60 °C und 80 °C eingestellt.

30 kg der nach Beispiel 1 bzw. 2 hergestellten und auf 50 % Trockensubstanzgehalt eingestellten Inulinhydrolysats werden auf die Trennsäule aufgetragen und mit entmineralisiertem Wasser eluiert.

Durch die Wahl eines geeigneten Schnittpunkts können mehr als 90 % der im Inulinhydrolysat vorliegenden homooligomeren Fructooligosaccharide in einer Fraktion mit einer Reinheit > 99 % erhalten werden.

Eine HPLC- bzw. gelchromatographische Analyse zeigt, daß die homooligomere Fructooligosaccharidfraktion neben Inulobiose nur Inulotriose, -tetraose, -pentaose, -hexaose und - heptaose enthält.

### Beispiel 4: Hydrierung

Die nach Beispiel 3 erhaltene homooligomere Fructoseoligosaccharidfraktion wird durch Eindampfung auf 40 % Trockensubstanzgehalt eingestellt.

450 ml dieser Lösung werden in einen Laborautoklaven in Gegenwart von Raney-Nickel mit Wasserstoff bei 150 bar und 80 °C in 18 h hydriert. Die hydrierte Lösung wird aus dem Autoklaven gepumpt, filtriert und durch Ionenaustausch gereinigt.

Diese gereinigte Lösung stellt bereits das erfindungsgemäße Süßungsmittel in flüssiger Form dar. Es kann durch eine der an sich bekannten Trocknungsmethoden, z.B. Sprühtrocknung in trockene Form überführt werden.

Die Hydrierung kann in Gegenwart anderer Katalysatoren sowohl batchweise als auch kontinuierlich durchgeführt werden und ist nicht auf die in diesem Beispiel beschriebene Methode beschränkt.

### Beispiel 5: Isolierung und Charakterisierung

Das nach Beispiel 4 erhaltene Süßungsmittel wurde zur Charakterisierung der Komponenten wie folgt aufgetrennt: Eine 100 cm³ Probe mit 15 % TS-Gehalt wurde auf eine mit Fraktogel® HW 40 S (Merck, Darmstadt) gefüllte und auf 54 °C temperierte Trennsäule (Durchmesser 0,1 m; Länge 3 m) aufgetragen und mit entsalztem Wasser mit einer Durchflußrate von 0,7 l·h⁻¹ eluiert. Durch geeignete Schnittpunkte konnten Fraktionen mit Reinheiten > 95 % gesammelt werden. Diese wurden durch erneute Chromatographie weiter gereinigt. Es wurden folgende Substanzen erhalten:
- 2-0-β-D-Fructofuranosyl-D-mannit,
- 2-0-β-D-Fructofuranosyl-D-sorbit,
- 2-0-(1-0-β-D-Fructofuranosyl)-β-D-fructofuranosyl-D-mannit,
- 2-0-(1-0-β-D-Fructofuranosyl)-β-D-fructofuranosyl-D-sorbit,
- [2-0-(1-0-β-D-Fructofuranosyl)]₂-β-D-fructofuranosyl-D-mannit,
- [2-0-(1-0-β-D-Fructofuranosyl)]₂-β-D-fructofuranosyl-D-sorbit,
und durch Massenspektrometrie und ¹³C-NMR-Spektroskopie nachgewiesen.

### Beispiel 6: Relative Süßkraft

In einem Dreiecktest wurden von jeweils 15 Prüfpersonen folgende Lösungen bei Raumtemperatur gegeneinander verglichen:
a) Zwei 7%ige Saccharoselösungen gegen eine 15,5%ige Lösung des neuen Süßungsmittels.
b) Zwei 7%ige Saccharoselösungen gegen eine 17,5%ige Lösung des neuen Süßungsmittels.
c) Zwei 7%ige Saccharoselösungen gegen eine 18,5%ige Lösung des neuen Süßungsmittels.

Beim Test a) wurde von 6 Personen das neue Süßungsmittel herausgefunden: Kein statistisch gesicherter Unterschied zu den Saccharoselösungen.

Beim Test b) wurde von 12 Personen das neue Süßungsmittel als "süßer" herausgefunden: Statistisch gesicherter Unterschied mit p = 0,99.

Beim Test c) wurde ebenfalls von 12 Personen das neue Süßungsmittel als "süßer" herausgefunden. Statistisch gesicherter Unterschied mit p = 0,99.

Die Süßkraft des erfindungsgemäßen Süßungsmittels beträgt 45 % der Saccharose. Zur Anhebung der Süßkraft läßt sich das neue Süßungsmittel mit Fructose, Xylit, Saccharin, Cyclamat, Aspartam oder Acesulfam-K mischen.

### Beispiel 7: Erdbeerkonfitüre

1 kg zerkleinerte Erdbeeren wurden, zusammen mit 1 kg des neuen Süßungsmittels und 8 g eines mittelveresterten Pektins mit 150 °SAG-USA (Ullmann, Enzyklopädie der technischen Chemie, 3. Auflage, Bd. 13, S. 180) sowie 7 g Weinsäure 3 Minuten lang gekocht und in vorbereitete Gläser abgefüllt.

Ein Vergleich mit einer mit der gleichen Menge Zucker hergestellten Konfitüre zeigte keinen signifikanten Unterschied bezüglich Konsistenz; erfindungsgemäß war die Süße etwas geringer dafür jedoch der Erdbeergeschmack spürbar stärker.

### Beispiel 8: Speiseeis

Ein Standard-Speiseeis mit Eiern, Vanille und Saccharose wurde nach folgendem Rezept hergestellt:

| | |
|---|---|
| Saccharose | 100 g |
| Teilentrahmte Milch | 500 ml |
| Magermilchpulver | 20 g |
| Invertzucker | 40 g |
| Apfelpektin | 1 g |
| Creme fraiche | 40 g |
| 3 Eigelb | |
| Eine halbe Vanilleschote | |

Zum Vergleich wurde ein Speiseeis hergestellt in dem die 100 g Saccharose durch 100 g des neuen Süßungsmittels und die 40 g Invertzucker durch 40 g Isomalt ersetzt wurden.

Gemäß einer Gruppe von 15 geschulten Testpersonen konnte kein signifikanter Unterschied zwischen den beiden Zubereitungen festgestellt werden. Alle Testpersonen stimmten überein, daß das Speiseeis mit dem neuen Süßungsmittel eine bessere Konsistenz aufweist.

### Beispiel 9: Kuchen

Ein Standard-Kuchen wurde nach folgender Rezeptur hergestellt:

| | |
|---|---|
| Saccharose | 20 g |
| Backhefe | 7,5 g |
| Mehl | 250 g |
| Salz | 5 g |
| Butter | 100 g |
| 2 Eier | |
| 4 Eßlöffel Wasser | |

Zum Vergleich wurde ein Kuchen hergestellt in dem die 20 g Saccharose durch 20 g des neuen Süßungsmittel ersetzt wurden.

Eine Gruppe von 15 geschulten Testpersonen konnte keine signifikanten Unterschiede zwischen den beiden Kuchen feststellen.

## Patentansprüche

1. Kalorienarmes, nicht-kariogenes und für Diabetiker geeignetes Süßungsmittel, **dadurch gekennzeichnet**, daß es aus (Fructosyl)ₙ-mannit- und/oder (Fructosyl)ₙ-sorbit, wobei n = 1-6 ist, oder einem Gemisch dieser Verbindungen besteht.

2. Verfahren zur Herstellung des Süßungsmittels nach Anspruch 1, **dadurch gekennzeichnet**, daß
a) man zunächst ein langkettiges Inulin mit einer kettenlänge > DP20 enzymatisch zu homooligomeren Fructooligosacchariden vom Typ F-Fₙ (Fructosylfructosetyp, n = 1-6) sowie heterooligomeren Fructooligosacchariden vom Typ G-Fn (Glucosylfructosetyp, n = 1-6) hydrolysiert,
b) durch chromatographische Trennung des Hydrolysats die homooligomeren Fructooligosaccharide (Typ F-Fn) von Fructose und den heterooligomeren Fructooligosacchariden abtrennt,
c) die homooligomeren Fructooligosaccharide hydriert und
d) das entstandene Gemisch durch an sich bekannte Methoden reinigt und in trockene Form überführt.

3. Verfahren nach Anspruch 2, **dadurch gekennzeichnet**, daß man das Inulin mit einer endo-Inulinase hydrolysiert.

4. Verfahren nach einem der Ansprüche 2 bis 3, **dadurch gekennzeichnet**, daß das Hydrolysat nach der enzymatischen Spaltung weitgehend reduzierende Fructooligosaccharide enthält.

5. Verfahren nach einem der Ansprüche 2 bis 4, **dadurch gekennzeichnet**, daß das Hydrolysat nach der enzymatischen Spaltung mindestens 75 % auf Trockensubstanzgehalt an reduzierenden Fructooligosacchariden enthält.

6. Verwendung des Süßungsmittels nach Anspruch 1 in Nahrungs- und Genußmitteln.

7. (Fructosyl)ₙ-mannit- und (Fructosyl)ₙ-sorbit-Gemisch, wobei n = 1-6 ist.

8. 2-0-β-D-Fructofuranosyl-D-mannit, 2-0-β-D-Fructofuranosyl-D-sorbit, 2-0-(1-0-β-D-Fructofuranosyl)-β-D-fructofuranosyl-D-mannit, 2-0-(1-0-β-D-Fructofuranosyl)-β-D-fructofuranosyl-D-sorbit [2-0-(1-0-β-D-Fructofuranosyl)]₂-β-D-fructofuranosyl-D-mannit, [2-0-(1-0-β-D-Fructafuranosyl)]₂-β-D-fructofuranosyl-D-sorbit.

## Claims

1. A low-calorie, non-cariogenic sweetener suitable for diabetics, characterised in that it comprises (fructosyl)ₙ-mannitol and/or (fructosyl)ₙ-sorbitol, where n = 1 - 6, or a mixture of these compounds.

2. A process for the preparation of the sweetener according to claim 1, characterised in that:
a) first a long-chain inulin with a chain length > DP20 is enzymatically hydrolysed to produce homo-oligomeric fructooligosaccharides of the F-Fn type (fructosyl-fructose type, n = 1 - 6) and hetero-oligomeric fructo-oligosaccharides of the G-Fn type (glucosyl-fructose type, n = 1 - 6);
b) the homo-oligomeric fructo-oligosaccharides (F-Fn type) are separated from fructose and the hetero-oligomeric fructooligosaccharides by chromatographic separation of the hydrolysate;
c) the homo-oligomeric fructo-oligosaccharides are hydrogenated, and
d) the resulting mixture is purified and dried by known methods.

3. A process according to claim 2, characterised in that the inulin is hydrolysed using an endo-inulinase.

4. A process according to either one of claims 2 and 3, characterised in that the hydrolysate substantially contains reducing fructo-oligosaccharides after enzymatic splitting.

5. A process according to any one of claims 2 to 4, characterised in that the hydrolysate has a dry substance content of reducing fructo-oligosaccharides of at least 75% after enzymatic splitting.

6. Use of the sweetener according to claim 1 in foodstuffs and luxury foods.

7. A (fructosyl)ₙ-mannitol and (fructosyl)ₙ-sorbitol mixture, where n = 1 - 6.

8. 2-0-β-D-fructofuranosyl- D-mannitol, 2-0-β-D-fructofuranosyl-D-sorbitol, 2-0-(1-0-β-D-fructofuranosyl)-β-D-fructofuranosyl-D-mannitol, 2-0-(1-0-β-D-fructofuranosyl)-β-D-fructofuranosyl-D-sorbitol, [2-0-(1-0-β-D-fructofuranosyl)]₂-β-D-fructofuranosyl-D-mannitol, [2-0-(1-0-β-D-fructofuranosyl)]₂-β-D-fructofuranosyl-D-sorbitol.

## Revendications

1. Edulcorant faiblement calorique, non cariogène et convenant pour les diabétiques, caractérisé en ce qu'il est constitué de (fructosyl)ₙ-mannitol et/ou de (fructosyl)ₙ-sorbitol, où n = 1 à 6, ou d'un mélange de ces composés.

2. Procédé de fabrication de l'édulcorant selon la revendication 1, caractérisé en ce que
a) on hydrolyse enzymatiquement une inuline à chaîne longue avec une longueur de chaîne > DP (degré de polymérisation) 20 en fructooligosaccharides homooligomères du type F-Fₙ (type fructosyl-fructose, n = 1 à 6) ainsi qu'en fructooligosaccharides hétérooligomères du type G-Fₙ (type glucosyl-fructose, n = 1 à 6),
b) on sépare par séparation chromatographique de l'hydrolysat les fructooligosaccharides homooligomères (type F-Fₙ) du fructose et des fructooligosaccharides hétérooligomères,
c) on hydrate les fructooligosaccharides homooligomères et
d) on purifie le mélange formé par des méthodes connues en elles-mêmes et on le convertit en forme sèche.

3. Procédé selon la revendication 2, caractérisé en ce qu'on hydrolyse l'inuline avec une endoinulinase.

4. Procédé selon l'une des revendications 2 à 3, caractérisé en ce que l'hydrolysat après la décomposition enzymatique contient principalement des fructooligosaccharides réducteurs.

5. Procédé selon l'une des revendications 2 à 4, caractérisé en ce que l'hydrolysat après la décomposition enzymatique contient au moins 75 % en teneur de substance sèche de fructooligosaccharides réducteurs.

6. Utilisation de l'édulcorant selon la revendication 1 dans des produits alimentaires et des stimulants.

7. Mélange de (fructosyl)ₙ-mannitol et de (fructosyl)ₙ-sorbitol, où n = 1 à 6.

8. 2-0-β-D-fructofuranosyl-D-mannitol, 2-0-β-D-fructofuranosyl-D-sorbitol, 2-0-(1-0-β-D-fructofuranosyl)-β-D-fructofuranosyl-D-mannitol, 2-0-(1-0-β-D-fructofuranosyl)-β-D-fructofuranosyl-D-sorbitol, [2-0-(1-0-β-D-fructofuranosyl)]₂-β-D-fructofuranosyl-D-mannitol, [2-0-(1-0-β-D-fructofuranosyl)]₂-β-D-fructofuranosyl-D-sorbitol.
